# EUROPEAN PATENT SPECIFICATION

(11) **EP 2 429 544 B1**
(45) Date of publication and mention of the grant of the patent: **20.04.2016**
(21) Application number: 10719333.6
(22) Date of filing: 07.05.2010
(51) Int. Cl.: A61K 35/74, A61P 29/00

(54) **SHORT-TIME HIGH TEMPERATURE TREATMENT GENERATES MICROBIAL PREPARATIONS WITH ANTI-INFLAMMATORY PROFILES**
Kurzzeitige Hochtemperaturbehandlung erzeugt mikrobielle Zubereitungen mit entzündungshemmenden Profilen
Traitement haute température de courte durée qui génère des préparations microbiennes avec des profils anti-inflammatoires

(30) Priority: 11.05.2009 EP 09159925
(43) Date of publication of application: 21.03.2012
(73) Proprietor: Nestec S.A., 1800 Vevey (CH)
(72) Inventor: PRIOULT, Guénolée, CH-1005 Lausanne (CH); MERCENIER, Annick, CH-1030 Bussigny (CH)
(74) Representative: Cogniat, Eric Jean Marie
(86) International application number: PCT/EP2010/056284
(87) International publication number: WO 2010/130659

(56) References cited:
- WO-A-2004/069156
- WO-A-2007/093619
- WO-A-2008/153377
- WO-A-2009/007515
- FR-A- 2 922 450
- US-A1- 2008 206 212

## Description

The present invention generally relates to the field of micro-organisms. In particular, the present invention concerns high temperature treated micro-organisms, e.g., probiotics and dairy starters, and applications of these bacteria. One embodiment of the present invention relates to high temperature treated micro-organisms, e.g., probiotics and dairy starters, and their use to prepare compositions to treat or prevent inflammatory disorders.

Probiotics are often defined as "live micro-organisms that when administered in adequate amounts confer health benefits to the host" (FAO/WHO Guidelines). Therefore, the vast majority of published literature deals with live probiotics. However, several studies investigated the health benefits delivered by non-replicating bacteria and most of them indicated that inactivation of probiotics, e.g. by heat treatment, leads to a loss of their purported health benefit (Rachmilewitz, D., et al., 2004, Gastroenterology 126:520-528; Castagliuolo, et al., 2005, FEMS Immunol.Med.Microbiol. 43:197-204; Gill, H. S. and K. J. Rutherfurd, 2001,Br.J.Nutr. 86:285-289; Kaila, M., et al., 1995, Arch.Dis.Child 72:51-53.). Some studies however showed that killed probiotics may retain some health effects (Rachmilewitz, D., et al., 2004, Gastroenterology 126:520-528; Gill, H. S. and K. J. Rutherfurd, 2001,Br.J.Nutr. 86:285-289).

The use of probiotics as a strategy to treat or prevent inflammatory bowel diseases has been reported in the literature and recently reviewed by Dotan et al. (Dotan, I. and D. Rachmilewitz. 2005; Curr.Opin.Gastroenterol. 21:426-430). For, example, a highly concentrated cocktail of eight live probiotic bacteria (VSL#3) has been shown to be effective in prevention (Gionchetti, P., et al., 2003, Gastroenterology 124:1202-1209) and treatment of recurrent or refractory pouchitis in humans (Gionchetti, P., et al., 2000, Gastroenterology 119:305-309; Mimura, T., et al., 2004, Gut 53:108-114). Interestingly using a murine model of DSS-induced colitis, Rachmilewitz et al. (Rachmilewitz, D., et al., 2004, Gastroenterology 126:520-528) reported that treatments with viable and γ-irradiated VSL#3 but not heat-killed VSL#3 protect against colitis. Similarly heat-killed *L. crispatus* failed to protect against DSS-induced colitis while its viable counterpart clearly reduced the loss of body weight and the MPO activity in the gut (Castagliuolo, et al., 2005, FEMS Immunol.Med.Microbiol. 43:197-204). These studies suggest that probiotics are more effective alive in the context of gut inflammation than their non-replicating counterparts.

Inactivated *L. reuteri* (heat-killed and γ-irradiated) was found not to be able to decrease the TNFα-induced IL-8 production by T84 cells while its live counterpart exhibited a significant beneficial effect (Ma, D., et al., 2004, Infect.Immun. 72:5308-5314).

Obviously, inactivated probiotics are much easier to handle, e.g., in the food industry, and/or to store. It is hence, encouraging to see that inactivated probiotics may have beneficial effects for the consumer, too, while - at the same time - it is disappointing to see, that these effects are usually found to be reduced or even abolished compared to viable probiotics.

The technologies described in the literature to render probiotic strains non-replicating are usually heat-treatment, γ-irradiation, UV light or chemical agents (formalin, paraformaldehyde). Hardly any studies have compared the effects of different inactivation methods on the biological function of the resulting non-replicating bacteria. In addition to the study of Rachmilewitz cited above, one such study reported that heat-treated and γ-irradiated bacteria induced different levels of cytokine secretion by epithelial cells *in vitro* (Wong, C. and Z. Ustunol. 2006, J.Food Prot. 69:2285-2288).

The technologies described and tested in the literature to render probiotic strains non-replicating are, however, in general not applicable or not easy to implement in industrial environments especially in the food industry.

Most products on the market today that contain probiotics are heat treated during their production. It would hence be convenient, to be able to heat treat probiotics either together with the produced product or at least in a similar way, while the probiotics retain or improve their beneficial properties or even gain a new beneficial property for the consumer.

However, heat treatments used to inactivate bacteria in the literature are generally long term treatments (from 20 min to 1 h or longer) at temperatures varying from 40 to 100°C that are not easy to implement at an industrial scale. Further, such heat treatments resulted generally in the art in an at least partial loss of probiotics activity.

In industrial scales no long term heat treatments are used but generally short term heat treatments, such as UHT-like heat treatments. This kind of heat treatment reduces bacterial loads, and reduces the processing time, thereby reducing the spoiling of nutrients.

It would hence be desirable to have available a microbial cell preparation obtainable from viable micro-organisms, e.g., probiotic bacteria or dairy starters, that can be heat treated in the same way as products typically are during the manufacturing process, and that generate or improve a health benefit such as preventing and/or treating inflammatory disorders.

It was consequently one object of the present invention, to provide the art with a composition comprising micro-organisms, e.g., probiotic bacteria or dairy starters, that can be industrially heat treated to reduce viable bacterial cells and that generates or improves a health benefit as a result of this heat treatment.

The present inventors were surprised to see that they could achieve this object by the subject matter of the independent claims. The dependent claims further develop the present invention.

The inventors demonstrate for the first time that micro-organisms, e.g., probiotic strains or dairy starters, heat treated by high temperatures for short times exhibit anti-inflammatory immune profiles regardless of their initial properties. In particular either a new anti-inflammatory profile is developed or an existing anti-inflammatory profile is enhanced by this heat treatment.

It is therefore now possible to generate non replicating micro-organisms with anti-inflammatory immune profiles by using specific heat treatment parameters that correspond to typical industrially applicable heat treatments, even if live counterparts are not anti-inflammatory strains.

This effect has been observed for example for several micro-organisms, e.g., for probiotic representatives of lactobacilli and bifidobacteria and also for dairy starter cultures.

Non-replicating probiotic micro-organisms have the advantage that they are far easier to handle than their live counterparts. Additionally, they are far more storage stable and need less stringent packaging conditions.

Non-replicating probiotic micro-organisms would allow developing a large variety of products, which by their nature do not allow the addition of live probiotics without additional measures to protect them. This plays a role for example in the provision of cereal bars, fruit juices, UHT-drinks, shelf stable drinks, etc.

Further, for example in severely immuno-compromised customers, the use of live probiotics may be limited in exceptional cases due to a potential risk to develop bacteremia. Here the inventors present a method to generate non-viable bacteria with anti-inflammatory profiles regardless of their initial immune profiles. Additionally, the provision of non-replicating probiotic micro-organisms allows the hot reconstitution, e.g., of powdered nutritional compositions while retaining health benefit for the consumer patient.

To the inventor's best knowledge, the use of a high temperature treatment for a short period of time in order to generate non-replicating bacteria with anti-inflammatory profiles has never been reported yet.

Consequently, the present invention is a composition comprising micro-organisms, wherein the micro-organisms were subjected to a high temperature treatment at 120 - 140°C for a short term of 5-15 seconds for use in the treatment or prevention of inflammatory disorders, wherein the micro-organisms are selected from the group consisting of probiotics, dairy starter cultures or combinations thereof, wherein the probiotics and/or dairy starters are selected from the group consisting of Bifidobacterium longum NCC 3001, Bifidobacterium longum NCC 2705, Bifidobacterium breve NCC 2950, Bifidobacterium lactis NCC 2818, Lactobacillus paracasei NCC 2461, Lactobacillus rhamnosus NCC 4007, Streptococcus thermophilus NCC 2019, Streptococcus thermophilus NCC 2059, Lactobacillus casei NCC 4006, Lactobacillus acidophilus NCC 3009, Lactobacillus casei ACA-DC 6002 (NCC 1825), Escherichia coli Nissle, Lactobacillus bulgaricus NCC 15, Lactococcus lactis NCC 2287, or combinations thereof.

The micro-organisms are preferably food grade micro-organisms. A micro-organism is food grade if it is approved for human or animal consumption.

In one embodiment, the micro-organisms may be probiotics.

Probiotics are defined for the purpose of the present invention as "Microbial cell preparations or components of microbial cells with a beneficial effect on the health or well-being of the host." (Salminen S, Ouwehand A. Benno Y. et al "Probiotics: how should they be defined" Trends Food Sci. Technol. 1999:10 107-10).

In a further embodiment, the micro-organisms may be dairy starter cultures.

The micro-organisms were subjected to a high temperature treatment at 120-140°C for a short term of 5-15 seconds.

This high temperature treatment renders the micro-organisms at least in part non-replicating.

"Non-replicating" micro-organisms include micro-organisms, e.g., probiotic bacteria and dairy starter cultures, which have been heat treated. This includes micro-organisms that are inactivated, dead, non-viable and/or present as fragments such as DNA, metabolites, cytoplasmic compounds, and/or cell wall materials.

"Non-replicating" means that no viable cells and/or colony forming units can be detected by classical plating methods. Such classical plating methods are summarized in the microbiology book: James Monroe Jay, Martin J. Loessner, David A. Golden. 2005. Modern food microbiology. 7th edition, Springer Science, New York, N.Y. 790 p. Typically, the absence of viable cells can be shown as follows: no visible colony on agar plates or no increasing turbidity in liquid growth medium after inoculation with different concentrations of bacterial preparations ('non replicating' samples) and incubation under appropriate conditions (aerobic and/or anaerobic atmosphere for at least 24h).

The high temperature treatment may be carried out at normal atmospheric pressure but may be also carried out under high pressure. Typical pressure ranges are form 1 to 50 bar, preferably from 1-10 bar, even more preferred from 2 to 5 bar. Obviously, it is preferred if the probiotics are heat treated in a medium that is either liquid or solid, when the heat is applied. An ideal pressure to be applied will therefore depend on the nature of the composition which the micro-organisms are provided in and on the temperature used.

The high temperature treatment is carried out in the temperature range of 120-140 °C.

The high temperature treatment is carried out for a short term of 5-15 seconds.

This given time frame refers to the time the micro-organisms, e.g. probiotics and dairy starter cultures, are subjected to the given temperature. Note that depending on the nature and amount of the composition the micro-organisms are provided in and depending on the architecture of the heating apparatus used, the time of heat application may differ.

Typically, however, the composition disclosed in the present invention and/or the micro-organisms are treated by a high temperature short time (HTST) treatment, flash pasteurization or a ultra high temperature (UHT) treatment.

A UHT treatment is Ultra-high temperature processing or a ultra-heat treatment (both abbreviated UHT) involving the at least partial sterilization of a composition by heating it for a short time, around 1-10 seconds, at a temperature exceeding 135°C (275°F), which is the temperature required to kill bacterial spores in milk. For example, processing milk in this way using temperatures exceeding 135° C permits a decrease of bacterial load in the necessary holding time (to 2-5 s) enabling a continuous flow operation.

There are two main types of UHT systems: the direct and indirect systems. In the direct system, products are treated by steam injection or steam infusion, whereas in the indirect system, products are heat treated using plate heat exchanger, tubular heat exchanger or scraped surface heat exchanger. Combinations of UHT systems may be applied at any step or at multiple steps in the process of product preparation. A HTST treatment is defined as follows (High Temperature/Short Time): Pasteurization method designed to achieve a 5-log reduction, killing 99,9999% of the number of viable micro-organisms in milk. This is considered adequate for destroying almost all yeasts, molds and common spoilage bacteria and also ensure adequate destruction of common pathogenic heat resistant organisms. In the HTST process milk is heated to 71.7°C (161°F) for 15-20 seconds.

Flash pasteurization is a method of heat pasteurization of perishable beverages like fruit and vegetable juices, beer and dairy products. It is done prior to filling into containers in order to kill spoilage micro-organisms, to make the products safer and extend their shelf life. The liquid moves in controlled continuous flow while subjected to temperatures of 71.5°C (160°F) to 74°C (165°F) for about 15 to 30 seconds.

For the purpose of the present disclosure the term "short time high temperature treatment" shall include high-temperature short time (HTST) treatments, UHT treatments, and flash pasteurization, for example.

Once rendered non-replicating by a high temperature treatment for a short time the resulting non-replicating probiotics will exhibit improved or new benefits as discussed above.

Any amount of non-replicating micro-organisms as discussed above will be effective. However, it is generally preferred, if at least 90 %, preferably, at least 95 %, more preferably at least 98 %, most preferably at least 99 %, ideally at least 99.9 %, most ideally all of the probiotics are non-replicating.

In one embodiment of the present invention all micro-organisms are non-replicating.

All micro-organisms may be used in the framework of the present disclosure.

Preferably, the micro-organisms are food-grade. Typical food-grade micro-organisms are probiotics.

Preferably, probiotics are used and may be selected from the group consisting of *Bifidobacterium, Lactobacillus, Lactococcus, Streptococcus, Candida,* or mixtures thereof.

For example the probiotics may be selected from the group consisting of *Bifidobacterium longum, Bifidobacterium lactis, Bifidobacterium animalis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus reuteri, Lactobacillus rhamnosus, Lactobacillus johnsonii, Lactobacillus plantarum, Lactobacillus fermentum, Lactococcus lactis,* and/or mixtures thereof.

The dairy starter cultures may be selected from the group consisting of *Propionibacterium, Streptococcus thermophilus, Lactococcus lactis, Lactococcus diacetylactis, Lactococcus cremoris, Lactobacillus bulgaricus, Lactobacillus helveticus, Lactobacillus delbrueckii,* and mixtures thereof.

It is essential that the high temperature treatment, e.g., at about 71.5-150 °C for a short time of about 1-120 seconds is carried out. This treatment may be applied to viable and/or to non-viable probiotics.

More than one high temperature treatment, e.g., at about 71.5-150 °C for a short time of about 1-120 seconds may be carried out.

In one disclosure the probiotics are selected from the group consisting of the genera *Bifidobacterium, Lactobacillus* and *Escherichia* or combinations thereof.

Typical examples of bifidobacteria are *Bifidobacterium longum, Bifidobacterium breve* or *Bifidobacterium lactis.* Typical examples of lactobacilli are *Lactobacillus paracasei, Lactobacillus casei, Lactobacillus acidophilus* or *Lactobacillus rhamnosus.* A typical example of *Escherichia* are *Escherichia coli* strains with a purported health benefit.

In one disclosure the dairy starter cultures are *Streptococcus thermophilus, Lactobacillus helveticus, Lactobacillus delbrueckii* and *Lactococcus lactis.*

The probiotics are selected from the group consisting of *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus casei* NCC 4006, *Lactobacillus casei* ACA-DC 6002 (NCC 1825), *Lactobacillus rhamnosus* NCC 4007, *Lactobacillus acidophilus* NCC 3009, *Escherichia coli* Nissle 1917, or combinations thereof.

Further, for example, the dairy starters are selected from the group consisting of *Streptococcus thermophilus* NCC 2019, *Streptococcus thermophilus* NCC 2059, *Lactobacillus delbrueckii* subsp. *bulgaricus* NCC 15 and *Lactococcus lactis* NCC 2287.

All strains have been deposited under the Budapest treaty as follows:

| | |
|---|---|
| *Bifidobacterium longum* NCC 3001: | ATCC BAA-999 |
| *Bifidobacterium longum* NCC 2705: | CNCM I-2618 |
| *Bifidobacterium breve* NCC 2950 | CNCM I-3865 |
| *Bifidobacterium lactis* NCC 2818: | CNCM I-3446 |
| *Lactobacillus paracasei* NCC 2461: | CNCM I-2116 |
| *Lactobacillus rhamnosus* NCC 4007: | CGMCC 1.3724 |
| *Streptococcus themophilus* NCC 2019: | CNCM I-1422 |
| *Streptococcus themophilus* NCC 2059: | CNCM I-4153 |
| *Lactococcus lactis* NCC 2287: | CNCM I-4154 |
| *Lactobacillus casei* NCC 4006: | CNCM I-1518 |
| *Lactobacillus casei* NCC 1825: | ACA-DC 6002 |
| *Lactobacillus acidophilus* NCC 3009: | ATCC 700396 |
| *Lactobacillus bulgaricus* NCC 15: | CNCM I-1198 |
| *Escherichia coli* Nissle 1917: | DSM 6601 |

The compositions disclosed in the present invention comprise short-time high temperature treated micro-organisms in an amount sufficient to at least partially treat inflammatory disorders and/or their complications. An amount adequate to accomplish this is defined as "a therapeutically effective dose". Amounts effective for this purpose will depend on a number of factors known to those of skill in the art such as the severity of the disease and the weight and general health state of the consumer, and on the effect of the food matrix.

In prophylactic applications, compositions disclosed in the invention are administered to a consumer susceptible to or otherwise at risk of inflammatory disorders in an amount that is sufficient to at least partially reduce the risk of developing inflammatory disorders. Such an amount is defined to be "a prophylactic effective dose". Again, the precise amounts depend on a number of patient specific factors such as the patient's state of health and weight, and on the effect of the food matrix.

Those skilled in the art will be able to adjust the therapeutically effective dose and/or the prophylactic effective dose appropriately.

In general the composition disclosed in the present invention contains "short-time high temperature" treated micro-organisms in a therapeutically effective dose and/or in a prophylactic effective dose.

Typically, the therapeutically effective dose and/or the prophylactic effective dose is in the range of about 0,005 mg - 1000 mg non-replicating, "short-time high temperature" treated micro-organisms per daily dose.

In terms of numerical amounts, the "short-time high temperature" treated non-replicating micro-organisms may be present in the composition in an amount corresponding to between 10⁴ and 10¹² equivalent cfu/g of the dry composition. Obviously, non-replicating micro-organisms do not form colonies, consequently, this term is to be understood as the amount of non replicating micro-organisms that is obtained from 10⁴ and 10¹² cfu/g replicating bacteria. This includes micro-organisms that are inactivated, non-viable or dead or present as fragments such as DNA or cell wall or cytoplasmic compounds. In other words, the quantity of micro-organisms which the composition contains is expressed in terms of the colony forming ability (cfu) of that quantity of micro-organisms as if all the micro-organisms were alive irrespective of whether they are, in fact, non replicating, such as inactivated or dead, fragmented or a mixture of any or all of these states.

Preferably the micro-organisms are present in an amount equivalent to between 10⁴ to 10⁹ cfu/g of dry composition, even more preferably in an amount equivalent to between 10⁵ and 10⁸ cfu/g of dry composition.

The composition disclosed in the present invention may be any kind of composition. The composition may be administered orally, enterally, parenterally (subcutaneously or intramuscularly), intra-vaginally, intra-rectally, topically or ocularly, for example. For example it may be a pharmaceutical composition, a nutraceutical, a food additive, a cosmetical composition, a pet food, a food product, or a drink.

Food products according to the present disclosure include dairy products, such as fermented milk products, e.g., yoghurts, buttermilk, etc; ice creams; concentrated milk; milk; dairy creams; flavoured milk drinks; whey based drinks; toppings; coffee creamers; chocolate; cheese based products; soups; sauces; purees; dressings; puddings; custards; baby foods; nutritional formulas, such as those for complete nutrition, for example for infants, children, teenagers, adults, the elderly or the critically ill; cereals and cereal bars, for example.

Drinks include for example milk- or yoghurt based drinks, fermented milk, protein drinks, coffee, tea, energy drinks, soy drinks, fruit and/or vegetable drinks, fruit and/or vegetable juices.

Cosmetical composition may include lotions, shampoos, eye drops, creams, for example.

A food additive or a medicament may be in the form of tablets; capsules; pastilles; sachets; gels; or liquids, e.g., nutritional solutions; for example.

The compositions may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. They may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, the compositions may contain an organic or inorganic carrier material suitable for oral or enteral administration as well as vitamins, minerals trace elements and other micronutrients in accordance with the recommendations of Government bodies such as the USRDA.

The composition disclosed in the present invention may further comprise other agents, depending on the intended use of the composition. For example a pain or fever relieving agent may be used to help minimize the uncomfortable feeling caused by the inflammatory disorder.

A stabilizing agent may be added to stabilize the composition and its constituents.

A flavouring agent and/or a colouring agent may be added to adjust flavours and to give the composition a colour that is easy to identify and/or that is perceived as pleasant.

Prebiotics may be added. Prebiotics may support the growth of probiotics before they are rendered non-replicating. Prebiotics may also act synergistically with viable probiotic bacteria that are present in the composition and/or that may be added.

"Prebiotic" means non-digestible food substances that promote the growth of health beneficial micro-organisms and/or probiotics in the intestines. They are not broken down in the stomach and/or upper intestine or absorbed in the Gl tract of the person ingesting them, but they are fermented by the gastrointestinal microbiota and/or by probiotics. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412.

The prebiotics that may be used in accordance with the present invention are not particularly limited and include all food substances that promote the growth of probiotics or health beneficial micro-organisms in the intestines. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides (FOS), galacto-oligosaccharides (GOS), isomalto-oligosaccharides (IMO), xylo-oligosaccharides (XOS), arabino-xylo oligosaccharides (AXOS), mannan-oligosaccharides (MOS), oligosaccharides of soy, glycosylsucrose (GS), lactosucrose (LS), lactulose (LA), palatinose-oligosaccharides (PAO), malto-oligosaccharides, gums and/or hydrolysates thereof, pectins and/or hydrolysates thereof.

A further probiotic may be added to the composition. All probiotic micro-organisms may be added additionally. Preferably, the probiotic may be selected for this purpose from the group consisting of *Bifidobacterium, Lactobacillus, Lactococcus, Enterococcus, Streptococcus, Kluyveromyces, Saccharoymces, Candida, Escherichia,* in particular selected from the group consisting of *Bifidobacterium longum, Bifidobacterium lactis, Bifidobacterium animalis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus lactis, Lactobacillus reuteri, Lactobacillus rhamnosus, Lactobacillus johnsonii, Lactobacillus plantarum, Lactobacillus salivarius, Lactococcus lactis, Enterococcus faecium, Saccharomyces cerevisiae, Saccharomyces boulardii, Escherichia coli* or mixtures thereof, preferably selected from the group consisting of *Bifidobacterium longum* NCC 3001 (ATCC BAA-999), *Bifidobacterium longum* NCC 2705 (CNCM I-2618), *Bifidobacterium longum* NCC 490 (CNCM I-2170), *Bifidobacterium lactis* NCC 2818 (C N C M I-3446), *Bifidobacterium breve* NCC 2950 (CNCM I-3865), *Lactobacillus paracasei* NCC 2461 (CNCM I-2116), *Lactobacillus johnsonii* NCC 533 (CNCM I-1225), *Lactobacillus rhamnosus GG* (ATCC53103), *Lactobacillus rhamnosus* NCC 4007 (CGMCC 1.3724), *Enterococcus faecium* SF68 (NCC 2768; NCIMB10415), and mixtures thereof.

The composition used in the present invention may be intended for any mammal, but is preferably intended for humans or pets.

The composition may also contain all vitamins and minerals understood to be essential in the daily diet and in nutritionally significant amounts. Minimum requirements have been established for certain vitamins and minerals. Examples of minerals, vitamins and other nutrients optionally present in the composition include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended consumer.

The composition of the present invention may contain at least on protein source, at least one carbohydrate source and at least one lipid source.

Any suitable dietary protein may be used, for example animal proteins (such as milk proteins, meat proteins and egg proteins); vegetable proteins (such as soy proteins, wheat proteins, rice proteins, and pea proteins); mixtures of free amino acids; or combinations thereof.

Milk proteins such as casein and whey, and soy proteins are particularly preferred. As far as whey proteins are concerned, the protein source may be based on acid whey or sweet whey or mixtures thereof and may include alpha- lactalbumin and beta-lactoglobulin in whatever proportions are desired.

Preferably however, in particular if the composition is an infant feeding formula, the protein source is based on modified sweet whey. Sweet whey is a readily available by-product of cheese making and is frequently used in the manufacture of infant formulas based on cows' milk.

The proteins may be intact or hydrolysed or a mixture of intact and hydrolysed proteins. It may be desirable to supply partially hydrolysed proteins (degree of hydrolysis between 2 and 20%).

If hydrolysed proteins are required, the hydrolysis process may be carried out as desired and as is known in the art. For example, a whey protein hydrolysate may be prepared by enzymatically hydrolysing the whey fraction in one or more steps.

If the composition of the present invention contains a protein source, then the amount of protein or protein equivalent in the composition is typically in the range of 1.6-7.5 g/100kcal of the composition.

In particular for nutritional formulas, the protein source should provide that the minimum requirements for essential amino acid content are met.

If the composition contains a carbohydrate source, the kind of carbohydrate to be used is not particularly limited. Any suitable carbohydrate may be used, for example sucrose, lactose, glucose, fructose, corn syrup solids, maltodextrins, starch and mixtures thereof. Combinations of different carbohydrate sources may be used. The carbohydrates may preferably provide 30% to 80% of the energy of the composition. For example, the composition may comprise a carbohydrate source in an amount of 9-18 g/100kcal of the composition.

If the composition contains a lipid source, the kind of lipid to be used is not particularly limited. If the composition includes a lipid source, the lipid source may provide 5% to 70% of the energy of the composition. Long chain n-3 and/or n-6 polyunsaturated fatty acids, such as DHA, ARA and/or EPA may be added. A suitable fat profile may be obtained using a blend of canola oil, corn oil, high-oleic acid sunflower oil and medium chain triglyceride oil. The composition may comprise a lipid source in an amount of 1.5-7 g/100kcal of the composition.

Dietary fibre may be added as well. They may be soluble or insoluble and in general a blend of the two types is preferred. Suitable sources of dietary fibre include soy, pea, oat, pectin, guar gum, arabic gum, fructooligosaccharides, galacto-oligosaccharides, sialyl-lactose and oligosaccharides derived from animal milks. A preferred fibre blend is a mixture of inulin with shorter chain fructo-oligosaccharides.

A composition of the present disclosure may be prepared by any manner known in the art. For example, if the composition is a nutritional formula, such as an infant feeding formula it may be prepared by blending together a protein source, a carbohydrate source, and a fat source in appropriate proportions. If used, emulsifiers may be included in the blend. Vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture.

The liquid mixture may then be thermally treated to reduce bacterial loads. For example, the liquid mixture may be rapidly heated to a temperature in the range of about 120°C to about 140°C for about 5 seconds to about 30 seconds. This may be carried out by steam injection or by heat exchanger; for example a plate heat exchanger.

The liquid mixture may then be cooled to about 60°C to about 85°C; for example by flash cooling. The liquid mixture may then be homogenised; for example in two stages at about 7 MPa to about 40 MPa in the first stage and about 2 MPa to about 14 MPa in the second stage. The homogenised mixture may then be further cooled to add any heat sensitive components; such as vitamins and minerals.

The pH and solids content of the homogenised mixture is conveniently standardised at this point. The homogenised mixture is transferred to a suitable drying apparatus such as a spray drier or freeze drier and converted to powder. The powder should have a moisture content of less than about 5% by weight.

The probiotics may be cultured according to any suitable method and prepared for addition to the nutritional composition by freeze-drying or spray-drying for example. The probiotics may then be added to the composition before the composition is heat treated to reduce bacterial loads. This will automatically render the probiotics at least in part non-replicating.

Alternatively, of course, the probiotics may also be short-time high temperature treated individually, and then added to the composition as non-replicating probiotics, e.g. in a liquid or powder form.

The selected probiotic(s) may be cultured according to any suitable method and prepared for addition to the composition by freeze-drying or spray-drying for example. Alternatively, bacterial preparations can be bought from specialist suppliers already prepared in a suitable form for addition to food products.

By analysing the immune profiles of live and "short-time high temperature" treated probiotics bacterial preparations, the inventors assessed the capacity of the probiotics to induce secretion of specific cytokines from human blood cells.

The immune profiles of viable probiotics were compared to the immune profiles of "short-time high temperature" treated cells. "Short-time high temperature" treated probiotics were found to induce different levels of cytokine secretion than their live counterparts.

The "short-time high temperature" treated probiotics induced less pro-inflammatory cytokines (TNF-α, IFN-γ, IL-12p40) while maintaining or inducing higher IL-10 secretion compared to their live counterparts. The resulting IL-12p40 / IL-10 ratios were lower for any "short-time high temperature" treated probiotics compared to live cells. By contrast, bacteria heat treated at 85°C for 20 min induced more pro-inflammatory cytokines and less IL-10 than live cells resulting in higher IL-12p40 / IL-10 ratios, demonstrating that the kind of heat treatment is essential for the present invention.

The IL-12p40 / IL-10 ratio obtained by incubating PBMC with probiotics *in vitro* is predictive of *in vivo* anti-inflammatory effects (Foligné, B., et al., 2007, World J.Gastroenterol. 13:236-243).

The present invention relates to the composition for use in treating or preventing inflammatory disorders.

Consequently, one embodiment is the use of a composition for the preparation of a product to treat or prevent inflammatory disorders.

The anti-inflammatory disorders that can be treated or prevented by the composition prepared by the use of the present invention are not particularly limited.

For example, they may be selected from the group consisting of acute inflammations such as sepsis; burns; and chronic inflammation, such as inflammatory bowel disease, e.g., Crohn's disease, ulcerative colitis, pouchitis; necrotizing enterocolitis; skin inflammation, such as UV or chemical-induced skin inflammation, eczema, reactive skin; irritable bowel syndrome; eye inflammation; allergy, asthma; obesity-associated inflammation; age-related low-grade inflammation, and combinations thereof.

The present invention also extends to a method to provide micro-organisms, e.g., probiotics and/or dairy starter cultures, in particular viable probiotics and/or viable dairy starter cultures with an anti-inflammatory effect or to improve the anti-inflammatory effect of micro-organisms, in particular of viable probiotics and/or viable dairy starter cultures, comprising the step of subjecting the micro-organisms to a high temperature treatment for a short time of at 120 - 140°C for a short term of 5-15 seconds.

If viable probiotics and/or dairy starter cultures are used in this method, the "short-time high temperature" treatment will result in rendering at least a part of the probiotics and/or dairy starter cultures non-replicating.

The "short-time high temperature" treatment may result in rendering at least 90 %, preferably, at least 95 %, more preferably at least 98 %, most preferably at least 99 %, ideally at least 99.9 %, most ideally all of the probiotics are non-replicating.

In a preferred embodiment of the present invention, the method comprises the step of adding viable probiotics to a composition and subjecting the probiotic containing composition to the "short-time high temperature" treatment.

The micro-organisms may also be heat treated before supplementation into products.

The composition may be any composition but is for example a food or nutritional product or a drink to be supplemented with probiotics.

In accordance with this the present invention also extends to a method to provide a composition comprising micro-organisms, e.g., probiotics and/or dairy starter cultures, preferably viable probiotics and/or viable dairy starter cultures, with anti-inflammatory properties or to improve its existing anti-inflammatory properties comprising the step of subjecting the micro-organisms to a high temperature treatment at at least 71.5 °C for at least 1 second, for example a high temperature treatment at about 71.5-150 °C for a short term of about 1-120 seconds.

This "short-time high temperature" treatment step can conveniently be carried out in industrial facilities, but can also be carried out at home using for example a steamer. This way, the anti-inflammatory effect could be imparted to a product directly prior to consumption.

Those skilled in the art will understand that they can freely combine all features of the present invention described herein, without departing from the scope of the invention as disclosed. In particular, features described for the uses and methods of the present invention may be applied to the composition and to the method of the present invention and vice versa.

Further advantages and features of the present invention are apparent from the following Examples and Figures.
Figures 1 A and B show the enhancement of the anti-inflammatory immune profiles of probiotics treated with "short-time high temperatures".
Figure 2 shows non anti-inflammatory probiotic strains that become anti-inflammatory, i.e. that exhibit pronounced anti-inflammatory immune profiles *in vitro* after being treated with "short-time high temperatures".
Figures 3 A and B show probiotic strains in use in commercially available products that exhibit enhanced or new anti-inflammatory immune profiles *in vitro* after being treated with "short-time high temperatures".
Figures 4 A and B show dairy starter strains (i.e. Lc1 starter strains) that exhibits enhanced or new anti-inflammatory immune profiles *in vitro* upon heat treatment at high temperatures.
Figure 5 shows a non anti-inflammatory probiotic strain that exhibits anti-inflammatory immune profiles *in vitro* after being treated with HTST treatments.
Figure 6: Principal Component Analysis on PBMC data (IL-12p40, IFN-γ, TNF-α, IL-10) generated with probiotic and dairy starter strains in their live and heat treated (140°C for 15 second) forms. Each dot represents one strain either live or heat treated identified by its NCC number or name.
Figure 7 shows IL-12p40 / IL-10 ratios of live and heat treated (85°C, 20min) strains. Overall, heat treatment at 85°C for 20 min leads to an increase of IL-12p40 / IL-10 ratios as opposed to "short-time high temperature" treatments of the present invention (Figures 1, 2, 3, 4 and 5).

### Examples:

### Methodology

### Bacterial preparations:

The health benefits delivered by live probiotics on the host immune system are generally considered to be strain specific. Probiotics inducing high levels of IL-10 and/or inducing low levels of pro-inflammatory cytokines *in vitro* (PBMC assay) have been shown to be potent anti-inflammatory strains *in vivo* (Foligné, B., et al., 2007, World J.Gastroenterol. 13:236-243).

Several probiotic strains were used to investigate the anti-inflammatory properties of heat treated probiotics. These were *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Lactobacillus casei* NCC 4006, *Lactobacillus acidophilus* NCC 3009, *Lactobacillus casei* ACA-DC 6002 (NCC 1825), and *Escherichia coli* Nissle. Several starter culture strains including some strains commercially used to produce Nestlé Lc1 fermented products were also tested: *Streptococcus thermophilus* NCC 2019, *Streptococcus thermophilus* NCC 2059, *Lactobacillus bulgaricus* NCC 15 and *Lactococcus lactis* NCC 2287.

Bacterial cells were cultivated in conditions optimized for each strain in 5-15L bioreactors. All typical bacterial growth media are usable. Such media are known to those skilled in the art. When pH was adjusted to 5.5, 30% base solution (either NaOH or Ca(OH)₂) was added continuously. When adequate, anaerobic conditions were maintained by gassing headspace with CO₂. *E. coli* was cultivated under standard aerobic conditions.

Bacterial cells were collected by centrifugation (5,000 x g, 4°C) and re-suspended in phosphate buffer saline (PBS) in adequate volumes in order to reach a final concentration of around 10⁹ -10¹⁰ cfu/ml. Part of the preparation was frozen at -80°C with 15% glycerol. Another part of the cells was heat treated by:
- Ultra High Temperature: 140°C for 15 sec; by indirect steam injection.
- High Temperature Short Time (HTST): 74°C, 90°C and 120°C for 15 sec by indirect steam injection
- Long Time Low Temperature (85°C, 20 min) in water bath

Upon heat treatment, samples were kept frozen at -80°C until use.

### In vitro immunoprofiling of bacterial preparations:

The immune profiles of live and heat treated bacterial preparations (i.e. the capacity to induce secretion of specific cytokines from human blood cells *in vitro*) were assessed. Human peripheral blood mononuclear cells (PBMCs) were isolated from blood filters. After separation by cell density gradient, mononuclear cells were collected and washed twice with Hank's balanced salt solution. Cells were then resuspended in Iscove's Modified Dulbecco's Medium (IMDM, Sigma) supplemented with 10% foetal calf serum (Bioconcept, Paris, france), 1% L-glutamine (Sigma), 1% penicillin/streptomycin (Sigma) and 0.1% gentamycin (Sigma). PBMCs (7x10⁵ cells/well) were then incubated with live and heat treated bacteria (equivalent 7x10⁶ cfu/well) in 48 well plates for 36h. The effects of live and heat treated bacteria were tested on PBMCs from 8 individual donors splitted into two separated experiments. After 36h incubation, culture plates were frozen and kept at -20°C until cytokine measurement. Cytokine profiling was performed in parallel (i.e. in the same experiment on the same batch of PBMCs) for live bacteria and their heat-treated counterparts.

Levels of cytokines (IFN-γ, IL-12p40, TNF-α and IL-10) in cell culture supernatants after 36h incubation were determined by ELISA (R&D DuoSet Human IL-10, BD OptEIA Human IL12p40, BD OptEIA Human TNFα, BD OptEIA Human IFN-γ) following manufacturer's instructions. IFN-γ, IL-12p40 and TNF-α are pro-inflammatory cytokines, whereas IL-10 is a potent anti-inflammatory mediator. Results are expressed as means (pg/ml) +/- SEM of 4 individual donors and are representative of two individual experiments performed with 4 donors each. The ratio IL-12p40 / IL-10 is calculated for each strain as a predictive value of *in vivo* anti-inflammatory effect (Foligné, B., et al., 2007, World J.Gastroenterol. 13:236-243).

Numerical cytokine values (pg/ml) determined by ELISA (see above) for each strain were transferred into BioNumerics v5.10 software (Applied Maths, Sint-Martens-Latem, Belgium). A Principal Component Analysis (PCA, dimensioning technique) was performed on this set of data. Subtraction of the averages over the characters and division by the variances over the characters were included in this analysis.

### Results

### Anti-inflammatory profiles generated by Ultra High Temperature (UHT) / High Temperature Short Time (HTST)-like treatments

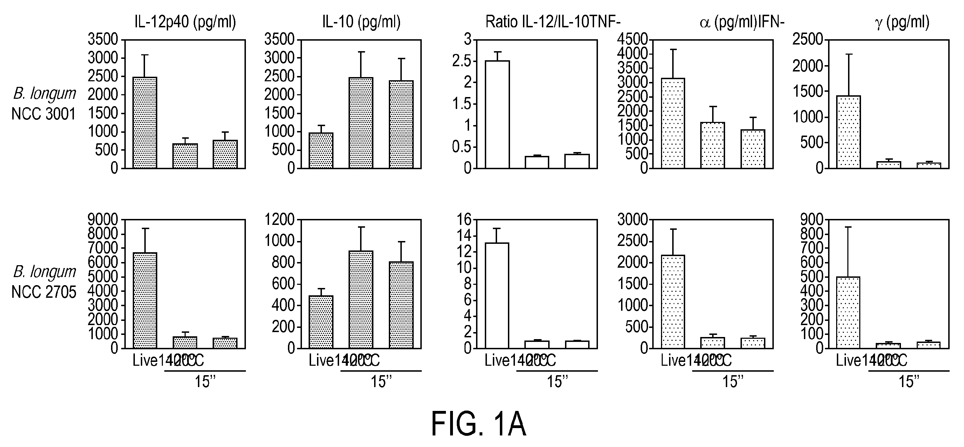

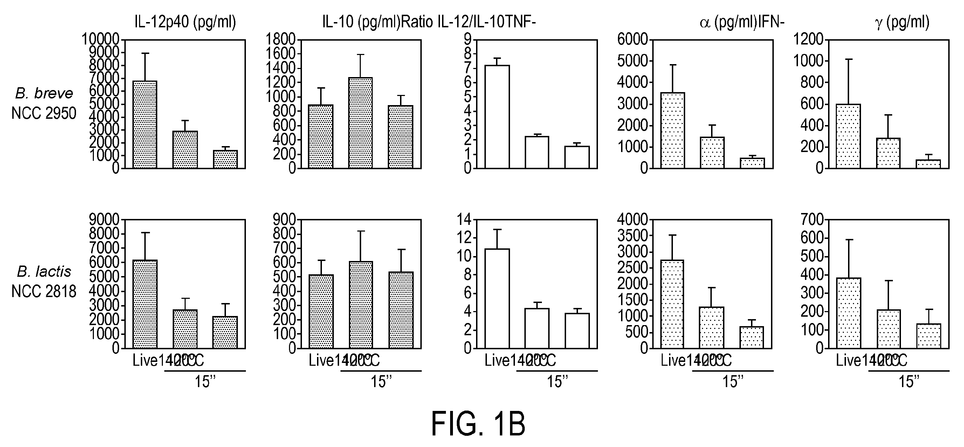

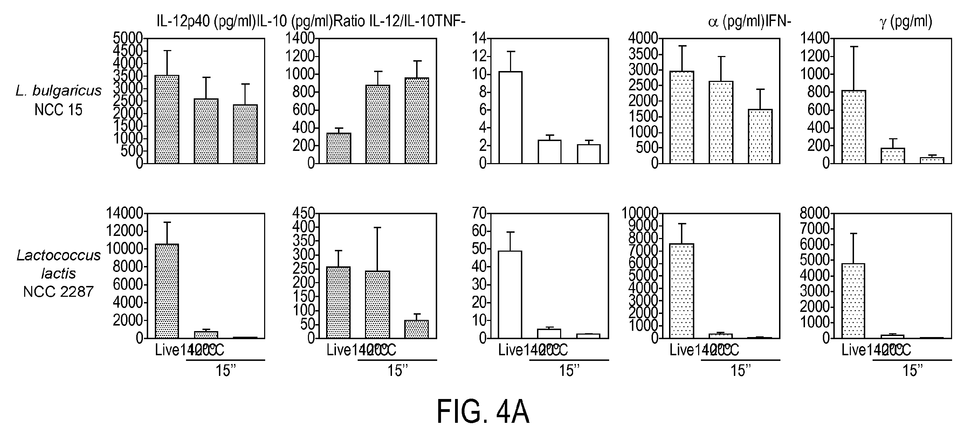

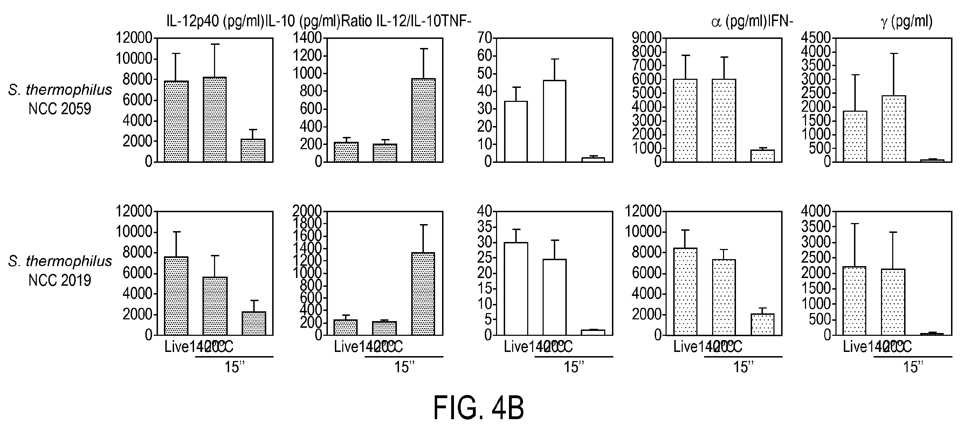

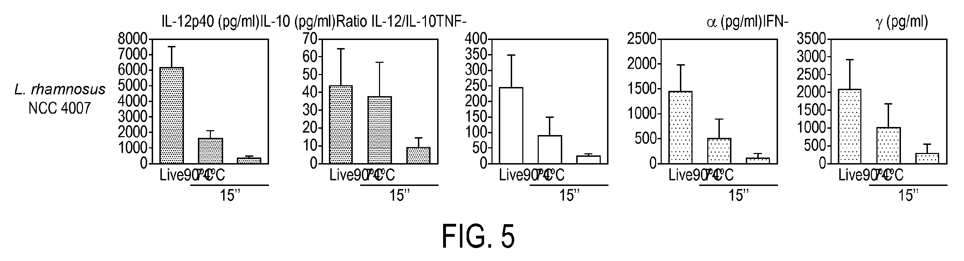

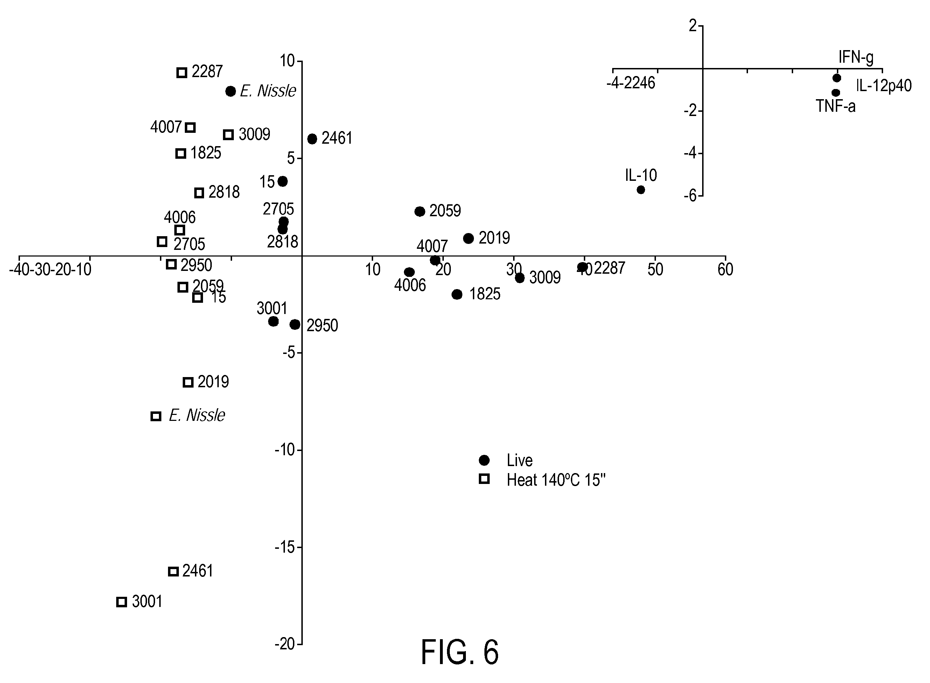

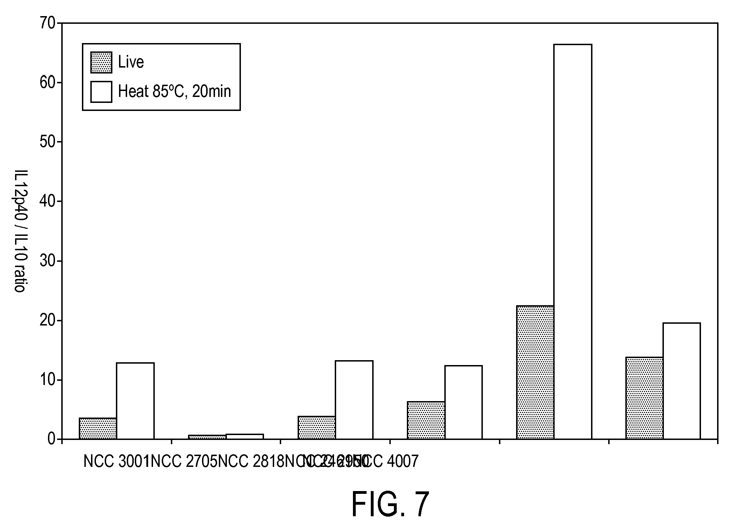

The probiotic strains under investigation were submitted to a series of heat treatments (Ultra High Temperature (UHT), High Temperature Short Time (HTST) and 85°C for 20 min) and their immune profiles were compared to those of live cells *in vitro.* Live micro-organisms (probiotics and/or dairy starter cultures) induced different levels of cytokine production when incubated with human PBMC (Figures 1, 2, 3, 4 and 5). Heat treatment of these micro-organisms modified the levels of cytokines produced by PBMC in a temperature dependent manner. "Short-time high temperature" treatments (120°C or 140°C for 15") generated non replicating bacteria with anti-inflammatory immune profiles (Figures 1, 2, 3 and 4). Indeed, UHT-like treated strains (140°C, 15 sec) induced less pro-inflammatory cytokines (TNF-α, IFN-y, IL-12p40) while maintaining or inducing additional IL-10 production (compared to live counterparts). The resulting IL-12p40 / IL-10 ratios were lower for any UHT-like treated strains compared to live cells (Figures 1, 2, 3 and 4). This observation was also valid for bacteria treated by HTST-like treatments, i.e. submitted to 120°C for 15 sec (Figures 1, 2, 3 and 4), or 74°C and 90°C for 15 sec (Figure 5). Heat treatments (UHT-like or HTST-like treatments) had a similar effect on *in vitro* immune profiles of probiotic strains (Figures 1, 2, 3 and 5) and dairy starter cultures (Figure 4). Principal Component Analysis on PBMC data generated with live and heat treated (140°C, 15") probiotic and dairy starter strains revealed that live strains are spread all along the x axis, illustrating that strains exhibit very different immune profiles *in vitro,* from low (left side) to high (right side) inducers of pro-inflammatory cytokines. Heat treated strains cluster on the left side of the graph, showing that pro-inflammatory cytokines are much less induced by heat treated strains (Figure 6). By contrast, bacteria heat treated at 85°C for 20 min induced more pro-inflammatory cytokines and less IL-10 than live cells resulting in higher IL-12p40 / IL-10 ratios (Figure 7).

Anti-inflammatory profiles are enhanced or generated by UHT-like and HTST-like treatments.

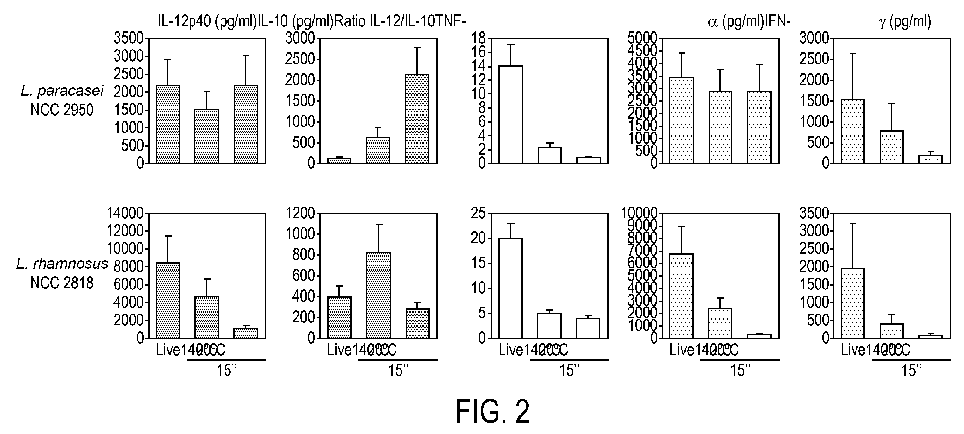

UHT and HTST treated strains exhibit anti-inflammatory profiles regardless of their respective initial immune profiles (live cells). Probiotic strains known to be anti-inflammatory *in vivo* and exhibiting anti-inflammatory profiles *in vitro* (*B. longum* NCC 3001, *B. longum* NCC 2705, *B. breve* NCC 2950, *B. lactis* NCC 2818) were shown to exhibit enhanced anti-inflammatory profiles *in vitro* after "short-time high temperature" treatments. As shown in Figure 1, the IL-12p40 / IL-10 ratios of UHT-like treated *Bifidobacterium* strains were lower than those from the live counterparts, thus showing improved anti-inflammatory profiles of UHT-like treated samples. More strikingly, the generation of anti-inflammatory profiles by UHT-like and HTST-like treatments was also confirmed for non anti-inflammatory live strains. Both live *L*. *rhamnosus* NCC 4007 and *L. paracasei* NCC 2461 exhibit high IL-12p40 / IL-10 ratios *in vitro* (Figures 2 and 5). The two live strains were shown to be not protective against TNBS-induced colitis in mice. The IL-12p40 / IL-10 ratios induced by *L. rhamnosus* NCC 4007 and *L. paracasei* NCC 2461 were dramatically reduced after "short-time high temperature" treatments (UHT or HTST) reaching levels as low as those obtained with *Bifidobacterium* strains. These low IL-12p40 / IL-10 ratios are due to low levels of IL-12p40 production combined with no change (*L*. *rhamnosus* NCC 4007) or a dramatic induction of IL-10 secretion (*L. paracasei* NCC 2461) (Figure 2).

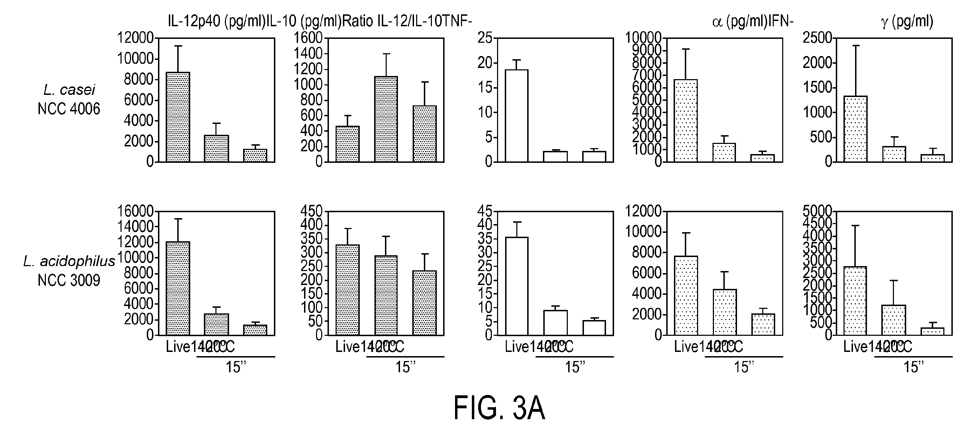

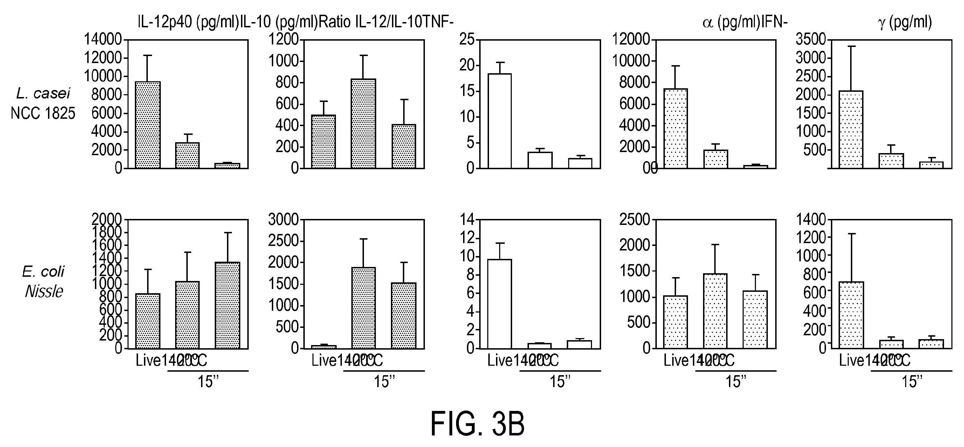

As a consequence:
- Anti-inflammatory profiles of live micro-organisms can be enhanced by UHT-like and HTST-like heat treatments (for instance *B. longum* NCC 2705, B. *longum* NCC 3001, *B*. *breve* NCC 2950, *B. lactis* NCC 2818)
- Anti-inflammatory profiles can be generated from non anti-inflammatory live micro-organisms (for example *L. rhamnosus* NCC 4007, *L. paracasei* NCC 2461, dairy starters S. *thermophilus* NCC 2019) by UHT-like and HTST-like heat treatments.
- Anti-inflammatory profiles were also demonstrated for strains isolated from commercially available products (Figures 3 A & B) including a probiotic *E. coli* strain.

The impact of UHT/HTST-like treatments was similar for all tested probiotics and dairy starters, for example lactobacilli, bifidobacteria and streptococci.

UHT/HTST-like treatments were applied to several lactobacilli, bifidobacteria and streptococci exhibiting different *in vitro* immune profiles. All the strains induced less pro-inflammatory cytokines after UHT/HTST-like treatments than their live counterparts (Figures 1, 2, 3, 4, 5 and 6) demonstrating that the effect of UHT/HTST-like treatments on the immune properties of the resulting non replicating bacteria can be generalized to all probiotics, in particular to lactobacilli and bifidobacteria and specific *E. coli* strains and to all dairy starter cultures in particular to streptococci, lactococci and lactobacilli.

| | | |
|---|---|---|
| **0-1** | Form **PCT/RO/134 (SAFE) Indications Relating to Deposited Microorganism(s) or Other Biological Material (PCT Rule 13bis)** | |
| 0-1-1 | **Prepared Using** | PCT Online Filing Version 3.5.000.204 MT/FOP 20020701/0.20.5.9 |
| **0-2** | **International Application No.** | PCT/EP2010/056284 |
| **0-3** | **Applicant's or agent's file reference** | NO 10156-WO |
| | | |
| **1** | **The indications made below relate to the deposited microorganism(s) or other biological material referred to in the description on:** | |
| **1-1** | **page** | 9 |
| **1-2** | **line** | 18 |
| **1-3** | **Identification of deposit** | |
| 1-3-1 | Name of depositary institution | CNCM Collection nationale de cultures de micro-organismes |
| 1-3-2 | Address of depositary institution | Institut Pasteur, 28, rue du Dr Roux, 75724 Paris Cedex 15, France |
| 1-3-3 | Date of deposit | 02 March 2001 (02.03.2001) |
| 1-3-4 | Accession Number | CNCM I-2618 |
| **1-5** | **Designated States for Which Indications are Made** | all designations |
| **2** | **The indications made below relate to the deposited microorganism(s) or other biological material referred to in the description on:** | |
| **2-1** | **page** | 9 |
| **2-2** | **line** | 19 |
| **2-3** | **Identification of deposit** | |
| 2-3-1 | Name of depositary institution | CNCM Collection nationale de cultures de micro-organismes |
| 2-3-2 | Address of depositary institution | Institut Pasteur, 28, rue du Dr Roux, 75724 Paris Cedex 15, France |
| 2-3-3 | Date of deposit | 10 January 2008 (10.01.2008) |
| 2-3-4 | Accession Number | CNCM I-3865 |
| 2-5 | **Designated States for Which Indications are Made** | all designations |
| **3** | **The indications made below relate to the deposited microorganism(s) or other biological material referred to in the description on:** | |
| **3-1** | **page** | 9 |
| **3-2** | **line** | 20 |
| **3-3** | **Identification of deposit** | |
| 3-3-1 | Name of depositary institution | CNCM Collection nationale de cultures de micro-organismes |
| 3-3-2 | Address of depositary institution | Institut Pasteur, 28, rue du Dr Roux, 75724 Paris Cedex 15, France |
| 3-3-3 | Date of deposit | 09 September 2005 (09.09.2005) |
| 3-3-4 | Accession Number | CNCM I-3446 |
| **3-5** | **Designated States for Which Indications are Made** | all designations |
| **4** | **The indications made below relate to the deposited microorganism(s) or other biological material referred to in the description on:** | |
| **4-1** | **page** | 9 |
| **4-2** | **line** | 21 |
| **4-3** | **Identification of deposit** | |
| 4-3-1 | Name of depositary institution | CNCM Collection nationale de cultures de micro-organismes |
| 4-3-2 | Address of depositary institution | Institut Pasteur, 28, rue du Dr Roux, 75724 Paris Cedex 15, France |
| 4-3-3 | Date of deposit | 12 February 1999 (12.02.1999) |
| 4-3-4 | Accession Number | CNCM I-2116 |
| **4-5** | **Designated States for Which Indications are Made** | all designations |
| **5** | **The indications made below relate to the deposited microorganism(s) or other biological material referred to in the description on:** | |
| **5-1** | **page** | 9 |
| **5-2** | **line** | 22 |
| **5-3** | **Identification of deposit** | |
| 5-3-1 | Name of depositary institution | CGMCC China General Microbiological Culture Collection Center |
| 5-3-2 | Address of depositary institution | China Committee for Culture Collection of Microorganisms, P.O. Box 2714, Beijing 100080, China |
| 5-3-3 | Date of deposit | 05 November 2004 (05.11.2004) |
| 5-3-4 | Accession Number | CGMCC 1.3724 |
| **5-5** | **Designated States for Which Indications are Made** | all designations |
| **6** | **The indications made below relate to the deposited microorganism(s) or other biological material referred to in the description on:** | |
| **6-1** | **page** | 9 |
| **6-2** | **line** | 29 |
| **6-3** | **Identification of deposit** | |
| 6-3-1 | Name of depositary institution | CNCM Collection nationale de cultures de micro-organismes |
| 6-3-2 | Address of depositary institution | Institut Pasteur, 28, rue du Dr Roux, 75724 Paris Cedex 15, France |
| 6-3-3 | Date of deposit | 20 May 1992 (20.05.1992) |
| 6-3-4 | Accession Number | CNCM I-1198 |
| **6-5** | **Designated States for Which Indications are Made** | all designations |
| **7** | **The indications made below relate to the deposited microorganism(s) or other biological material referred to in the description on:** | |
| **7-1** | **page** | 9 |
| **7-2** | **line** | 23 |
| **7-3** | **Identification of deposit** | |
| 7-3-1 | Name of depositary institution | CNCM Collection nationale de cultures de micro-organismes |
| 7-3-2 | Address of depositary institution | Institut Pasteur, 28, rue du Dr Roux, 75724 Paris Cedex 15, France |
| 7-3-3 | Date of deposit | 10 June 1994 (10.06.1994) |
| 7-3-4 | Accession Number | CNCM I-1422 |
| **7-5** | **Designated States for Which Indications are Made** | all designations |

### FOR RECEIVING OFFICE USE ONLY

| | | |
|---|---|---|
| **0-4** | **This form was received with the international application:** (yes or no) | YES |
| 0-4-1 | Authorized officer | Buffet, Lionel |

### FOR INTERNATIONAL BUREAU USE ONLY

| | | |
|---|---|---|
| **0-5** | **This form was received by the international Bureau on:** | |
| 0-5-1 | Authorized officer | |

## Claims

1. Composition comprising micro-organisms, wherein the micro-organisms were subjected to a high temperature treatment at 120 - 140°C for a short term of 5-15 seconds for use in the treatment or prevention of inflammatory disorders, wherein the micro-organisms are selected from the group consisting of probiotics, dairy starter cultures or combinations thereof, wherein the probiotics and/or dairy starters are selected from the group consisting of *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Streptococcus thermophilus* NCC 2019, *Streptococcus thermophilus* NCC 2059, *Lactobacillus casei* NCC 4006, *Lactobacillus acidophilus* NCC 3009, *Lactobacillus casei* ACA-DC 6002 (NCC 1825), *Escherichia coli* Nissle, *Lactobacillus bulgaricus* NCC 15, *Lactococcus lactis* NCC 2287, or combinations thereof.

2. Composition for use in accordance with claim 1wherein at least 90 %, preferably, at least 95 %, more preferably at least 98 %, most preferably at least 99 %, ideally at least 99.9 %, most ideally all of the probiotics are non-replicating.

3. Composition for use in accordance with one of the preceding claims wherein the composition may be to be administered orally, enterally, parenterally, e.g., subcutaneously or intramuscularly, intra-vaginally, intra-rectally, topically or ocularly, for example.

4. Composition for use in accordance with one of the preceding claims wherein the composition is intended for humans or pets.

5. Composition for use in accordance with one of the preceding claims wherein the composition contains about 0,005 mg - 1000 mg non-replicating micro-organisms per daily dose.

6. Composition for use in accordance with one of the preceding claims, wherein the inflammatory disorder is selected from the group consisting of acute inflammations such as sepsis; burns; and chronic inflammation, such as inflammatory bowel disease, e.g., Crohn's disease, ulcerative colitis, pouchitis; necrotizing enterocolitis; irritable bowel syndrome; skin inflammation, such as UV or chemical-induced skin inflammation, eczema, reactive skin; eye inflammation; allergy, asthma; obesity-associated inflammation; age-related low-grade inflammation, and combinations thereof.

7. Method to provide probiotics and/or dairy starter cultures, with an anti-inflammatory effect or to improve an anti-inflammatory effect of probiotics and/or dairy starter cultures, comprising the step of subjecting the micro-organisms to a high temperature treatment at 120 - 140°C for a short term of 5-15 seconds, wherein the probiotics and/or dairy starters are selected from the group consisting of *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Streptococcus thermophilus* NCC 2019, *Streptococcus thermophilus* NCC 2059, *Lactobacillus casei* NCC 4006, *Lactobacillus acidophilus* NCC 3009, *Lactobacillus casei* ACA-DC 6002 (NCC 1825), *Escherichia coli* Nissle, *Lactobacillus bulgaricus* NCC 15, *Lactococcus lactis* NCC 2287, or combinations thereof.

8. Method in accordance with claim 7, wherein the "short-time high temperature" treatment results in rendering at least at least 90 %, preferably, at least 95 %, more preferably at least 98 %, most preferably at least 99 %, ideally at least 99.9 %, most ideally all of the probiotics and/or dairy starter cultures being non-replicating.

9. Method in accordance with one of claims 7-8, comprising the step of adding viable probiotics and/or dairy starter cultures to a composition and subjecting the composition containing probiotics and/or dairy starter cultures to the "short-time high temperature" treatment at 120 - 140°C for a short term of 5-15 seconds.

10. Method to provide a composition comprising probiotics and/or dairy starter cultures, with anti-inflammatory properties or to improve its existing anti-inflammatory properties comprising the step of subjecting the probiotics and/or dairy starter cultures to a high temperature treatment at 120 - 140°C for a short term of 5-15 seconds, wherein the probiotics and/or dairy starters are selected from the group consisting of *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Streptococcus thermophilus* NCC 2019, *Streptococcus thermophilus* NCC 2059, *Lactobacillus casei* NCC 4006, *Lactobacillus acidophilus* NCC 3009, *Lactobacillus casei* ACA-DC 6002 (NCC 1825), *Escherichia coli* Nissle, *Lactobacillus bulgaricus* NCC 15, *Lactococcus lactis* NCC 2287, or combinations thereof.

## Patentansprüche

1. Zusammensetzung, die Mikroorganismen umfasst, wobei die Mikroorganismen über einen kurzen Zeitraum von 5-15 Sekunden einer Hochtemperaturbehandlung bei 120-140 °C unterzogen wurden, zur Verwendung bei der Behandlung oder Verhütung von entzündlichen Krankheiten, wobei die Mikroorganismen ausgewählt sind aus der Gruppe bestehend aus Probiotika, Milchsäurestarterkulturen oder Kombinationen davon, wobei die Probiotika und/oder Milchsäurestarter ausgewählt sind aus der Gruppe bestehend aus *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Streptococcus thermophilus* NCC 2019, *Streptococcus thermophilus* NCC 2059, *Lactobacillus casei* NCC 4006, *Lactobacillus acidophilus* NCC 3009, *Lactobacillus casei* ACA-DC 6002 (NCC 1825), *Escherichia coli* Nissle, *Lactobacillus bulgaricus* NCC 15, *Lactococcus lactis* NCC 2287 oder Kombinationen davon.

2. Zusammensetzung zur Verwendung gemäß Anspruch 1, wobei mindestens 90 %, vorzugsweise mindestens 95 %, mehr bevorzugt mindestens 98 %, am meisten bevorzugt mindestens 99 %, idealerweise mindestens 99,9 %, bestenfalls alle Probiotika replikationsunfähig sind.

3. Zusammensetzung zur Verwendung gemäß einem der vorangehenden Ansprüche, wobei die Zusammensetzung beispielsweise oral, enteral, parenteral, z.B. subkutan oder intramuskulär, intravaginal, intrarektal, topisch oder okular verabreicht werden kann.

4. Zusammensetzung zur Verwendung gemäß einem der vorangehenden Ansprüche, wobei die Zusammensetzung für Menschen oder Haustiere gedacht ist.

5. Zusammensetzung zur Verwendung gemäß einem der vorangehenden Ansprüche, wobei die Zusammensetzung etwa 0,005 mg - 1000 mg replikationsunfähige Mikroorganismen pro Tagesdosis enthält.

6. Zusammensetzung zur Verwendung gemäß einem der vorangehenden Ansprüche, wobei die entzündliche Erkrankung ausgewählt ist aus der Gruppe bestehend aus akuten Entzündungen wie Sepsis; Verbrennungen; und chronischer Entzündung, wie entzündlicher Darmerkrankung, z.B. Morbus Crohn, Colitis ulcerosa, Pouchitis; nekrotisierender Enterokolitis; Reizdarmsyndrom; Hautentzündung, wie UV- oder chemisch induzierter Hautentzündung, Ekzem, überempfindlicher Haut, Augenentzündung; Allergie, Asthma, mit Adipositas assoziierter Entzündung; altersabhängiger leichter Entzündung und Kombinationen davon.

7. Verfahren, um Probiotika und/oder Milchsäurestarterkulturen mit einer entzündungshemmenden Wirkung anzubieten oder um eine entzündungshemmende Wirkung von Probiotika und/oder Milchsäurestarterkulturen zu verbessern, das den Schritt der Anwendung einer Hochtemperaturbehandlung bei 120-140 °C über einen kurzen Zeitraum von 5-15 Sekunden auf die Mikroorganismen umfasst, wobei die Probiotika und/oder Milchsäurestarter ausgewählt sind aus der Gruppe bestehend aus *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Streptococcus thermophilus* NCC 2019, *Streptococcus thermophilus* NCC 2059, *Lactobacillus casei* NCC 4006, *Lactobacillus acidophilus* NCC 3009, *Lactobacillus casei* ACA-DC 6002 (NCC 1825), *Escherichia coli* Nissle, *Lactobacillus bulgaricus* NCC 15, *Lactococcus lactis* NCC 2287 oder Kombinationen davon.

8. Verfahren gemäß Anspruch 7, wobei die "Kurzzeit-Hochtemperatur"-Behandlung dazu führt, dass mindestens 90 %, vorzugsweise mindestens 95 %, mehr bevorzugt mindestens 98 %, am meisten bevorzugt mindestens 99 %, idealerweise mindestens 99,9 %, bestenfalls alle Probiotika und/oder Milchsäurestarterkulturen replikationsunfähig sind.

9. Verfahren gemäß einem der Ansprüche 7-8, das den Schritt der Zugabe lebensfähiger Probiotika und/oder Milchsäurestarterkulturen zu einer Zusammensetzung und des Aussetzens der Probiotika und/oder Milchsäurestarterkulturen enthaltenden Zusammensetzung der "Kurzzeit-Hochtemperatur"-Behandlung bei 120 -140 °C über einen kurzen Zeitraum von 5-15 Sekunden umfasst.

10. Verfahren, um eine Zusammensetzung anzubieten, die Probiotika und/oder Milchsäurestarterkulturen mit entzündungshemmenden Eigenschaften umfasst, oder um deren vorhandene entzündungshemmenden Eigenschaften zu verbessern, das den Schritt des Aussetzens der Probiotika und/oder Milchsäurestarterkulturen einer Hochtemperaturbehandlung bei 120-140 °C über einen kurzen Zeitraum von
5-15 Sekunden umfasst, wobei die Probiotika und/oder Milchsäurestarter ausgewählt sind aus der Gruppe bestehend aus *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Streptococcus thermophilus* NCC 2019, *Streptococcus thermophilus* NCC 2059, *Lactobacillus casei* NCC 4006, *Lactobacillus acidophilus* NCC 3009, *Lactobacillus casei* ACA-DC 6002 (NCC 1825), *Escherichia coli* Nissle, *Lactobacillus bulgaricus* NCC 15, *Lactococcus lactis* NCC 2287 oder Kombinationen davon.

## Revendications

1. Composition comprenant des micro-organismes, dans laquelle les micro-organismes ont été soumis à un traitement à haute température à des températures de 120 à 140 °C pendant une courte période de 5 à 15 secondes, pouvant être utilisée pour le traitement ou la prévention de troubles inflammatoires, où les micro-organismes sont choisis dans le groupe constitué de probiotiques, cultures de ferment lactique ou leurs combinaisons, où les probiotiques et/ou les ferments lactiques sont choisis dans le groupe constitué de *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Streptococcus thermophilus* NCC 2019, *Streptococcus thermophilus* NCC 2059, *Lactobacillus casei* NCC 4006, *Lactobacillus acidophilus* NCC 3009, *Lactobacillus casei* ACA-DC 6002 (NCC 1825), *Escherichia coli* Nissle, *Lactobacillus bulgaricus* NCC 15, *Lactococcus lactis* NCC 2287 ou leurs combinaisons.

2. Composition utilisable selon la revendication 1, où au moins 90 %, de préférence au moins 95 %, plus préférablement au moins 98 %, le plus préférablement au moins 99 %, idéalement au moins 99,9 %, de la manière la plus idéale tous les probiotiques sont non réplicatifs.

3. Composition utilisable selon l'une des revendications précédentes, où la composition peut être destinée à être administrée par voie orale, par voie entérale, par voie parentérale, par exemple, par voie sous-cutanée ou par voie intramusculaire, par voie intravaginale, par voie intrarectale, par voie topique ou par voie oculaire, par exemple.

4. Composition utilisable selon l'une des revendications précédentes, où la composition est prévue pour des humains ou des animaux domestiques.

5. Composition utilisable selon l'une des revendications précédentes, où la composition contient environ 0,005 mg à 1000 mg de micro-organismes non réplicatifs par dose quotidienne.

6. Composition utilisable selon l'une des revendications précédentes, où le trouble inflammatoire est choisi dans le groupe constitué d'inflammations aiguës telles qu'une septicémie ; des brûlures ; et une inflammation chronique, telle qu'une maladie inflammatoire de l'intestin, par exemple, la maladie de Crohn, la rectocolite hémorragique, la pochite ; l'entérocolite nécrosante ; le syndrome du côlon irritable ; une inflammation de la peau, telle qu'une inflammation de la peau induite par les UV ou une substance chimique, l'eczéma, une peau sensible ; une inflammation de l'oeil ; une allergie, de l'asthme ; une inflammation associée à l'obésité ; une inflammation de faible intensité liée à l'âge et leurs combinaisons.

7. Procédé pour fournir des probiotiques et/ou des cultures de ferment lactique avec un effet anti-inflammatoire ou pour améliorer un effet anti-inflammatoire de probiotiques et/ou de cultures de ferment lactique, comprenant l'étape consistant à soumettre les micro-organismes à un traitement à haute température à des températures de 120 à 140 °C pendant une courte période de 5 à 15 secondes, où les probiotiques et/ou ferments lactiques sont choisis dans le groupe constitué de *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Streptococcus thermophilus* NCC 2019, *Streptococcus thermophilus* NCC 2059, *Lactobacillus casei* NCC 4006, *Lactobacillus acidophilus* NCC 3009, *Lactobacillus casei* ACA-DC 6002 (NCC 1825), *Escherichia coli* Nissle, *Lactobacillus bulgaricus* NCC 15, *Lactococcus lactis* NCC 2287 ou leurs combinaisons.

8. Procédé selon la revendication 7, où le traitement « à haute température pendant une courte période » a pour résultat de rendre non réplicatifs au moins 90 %, de préférence au moins 95 %, plus préférablement au moins 98 %, le plus préférablement au moins 99 %, idéalement au moins 99,9 %, de la manière la plus idéale tous les probiotiques et/ou cultures de ferment lactique.

9. Procédé selon l'une des revendications 7 à 8, comprenant l'étape consistant à ajouter des probiotiques et/ou cultures de ferment lactique viables à une composition et à soumettre la composition contenant des probiotiques et/ou des cultures de ferment lactique au traitement « à haute température pendant une courte période » à une température de 120 à 140 °C pendant une courte période de 5 à 15 secondes.

10. Procédé pour fournir une composition comprenant des probiotiques et/ou des cultures de ferment lactique avec des propriétés anti-inflammatoires ou pour améliorer ses propriétés anti-inflammatoires existantes comprenant l'étape consistant à soumettre les probiotiques et/ou cultures de ferment lactique à un traitement à haute température à une température de 120 à 140 °C pendant une courte période de 5 à 15 secondes, où les probiotiques et/ou ferments lactiques sont choisis dans le groupe constitué de *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Streptococcus thermophilus* NCC 2019, *Streptococcus thermophilus* NCC 2059, *Lactobacillus casei* NCC 4006, *Lactobacillus acidophilus* NCC 3009, *Lactobacillus casei* ACA-DC 6002 (NCC 1825), *Escherichia coli* Nissle, *Lactobacillus bulgaricus* NCC 15, *Lactococcus lactis* NCC 2287 ou leurs combinaisons.
